(19) Europäisches Patentamt
European Patent Office
Office européen des brevets

(11) **EP 2 831 020 B1**

(12) **FASCICULE DE BREVET EUROPEEN**

(45) Date de publication et mention de la délivrance du brevet:
**05.09.2018 Bulletin 2018/36**

(21) Numéro de dépôt: **13719583.0**

(22) Date de dépôt: **25.03.2013**

(51) Int Cl.:
*C07C 1/24* (2006.01)          *C07C 5/27* (2006.01)
*B01J 21/12* (2006.01)         *C07C 11/08* (2006.01)
*C07C 11/09* (2006.01)         *B01J 35/10* (2006.01)

(86) Numéro de dépôt international:
**PCT/FR2013/050630**

(87) Numéro de publication internationale:
**WO 2013/144491 (03.10.2013 Gazette 2013/40)**

(54) **PROCÉDÉ DE DÉSHYDRATATION ET D'ISOMÉRISATION D'ALCOOLS UTILISANT UN SOLIDE DE TYPE ALUMINOSILICATE NON ZÉOLITHIQUE**

VERFAHREN ZUR ENTWÄSSERUNG UND ISOMERISIERUNG VON ALKOHOLEN UNTER VERWENDUNG EINES ZEOLITHFREIEN ALUMINOSILIKATFESTSTOFFES

METHOD FOR DEHYDRATING AND ISOMERISING ALCOHOLS USING A NON-ZEOLITE ALUMINOSILICATE SOLID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.03.2012 FR 1200951**

(43) Date de publication de la demande:
**04.02.2015 Bulletin 2015/06**

(73) Titulaire: **IFP Energies nouvelles
92500 Rueil-Malmaison (FR)**

(72) Inventeurs:
• **CHAUMONNOT, Alexandra
69003 Lyon (FR)**
• **COUPARD, Vincent
69100 Villeurbanne (FR)**
• **MAURY, Sylvie
69440 Saint Maurice d'Argoire (FR)**

(56) Documents cités:
EP-A1- 0 659 719          WO-A1-2011/113834
FR-A1- 2 884 827

• **R.A. COMELLI ET AL: "Transformation of C1C4 Alcohols into Hydrocarbons on an Amorphous Silica-Alumina Catalyst", APPLIED CATALYSIS, vol. 36, 1 janvier 1988 (1988-01-01), pages 299-306, XP055046369, ISSN: 0166-9834, DOI: 10.1016/S0166-9834(00)80123-2**

## Description

### Domaine technique de l'invention

La présente invention concerne un procédé amélioré de production d'alcènes en $C_4$ ou butènes à partir d'une charge d'alcool. La charge d'alcool utilisée peut être obtenue par des procédés chimiques ou par des procédés fermentaires à partir de carbohydrates. Ce procédé met en oeuvre un catalyseur à base d'un solide de type aluminosilicate non zéolithique présentant des propriétés texturales et d'acidité particulières.

Les alcènes obtenus, et en particulier l'isobutène et le butène-1, présentent un intérêt important dans le domaine de l'industrie pétrochimique et de la synthèse organique, l'isobutène étant un composé clé dans la chimie des grands intermédiaires.

### Art antérieur

La déshydratation des monoalcools en $C_4$ a été étudiée depuis de nombreuses années, principalement comme voie de synthèse ou de purification de l'isobutène. Des catalyseurs à base d'acide minéral, de sel $FeCl_2$, d'oxydes métalliques comme l'alumine ou des zéolithes ont été utilisés pour cette application. On citera l'article de Adkins et al., J. Am. Chem. Soc. 1925, 47, 1163, où il est noté que l'alumine, après activation sous air, est efficace pour une courte durée en déshydratation de nombreux alcools. Les auteurs notent que seule l'activation sous air permet de rendre l'alumine active sur cette application.

On citera également l'article de Makarova et al., J. Catal., 1994, 149, 36, où l'on enseigne que la zéolithe MFI (ZSM-5) et les silices alumines amorphes sont également actives en déshydratation des butanols linéaires. L'isomérisation de position des butènes formée est mentionnée. Cependant, aucune isomérisation squeletalle n'est mise en évidence. La formation d'éthers (dibutyl éthers) est par contre observée.

On citera aussi les travaux de P. Berteau et al. décrits dans Applied Catalysis, 1991, 70, 307, qui ont étudié les propriétés acido-basiques de silices alumines à teneurs variables en Si et Al. Les propriétés acido-basiques variables ont été corrélées aux propriétés catalytiques dans la réaction de déshydratation du 1-butanol. Les auteurs concluent que sur les solides riches en alumine (faible acidité de Brönsted), seule la déshydratation du 1-butanol en butène-1 ainsi que la formation de l'éther (dibutyléther) ont lieu. Par contre, sur les solides riches en silice (acidité de Brönsted exacerbée), la déshydratation en butène-1 et l'isomérisation rapide de position en butène-2 cis et trans a lieu. Aucune réaction d'isomérisation squeletalle n'est observée.

Le brevet US 5.545.793 décrit un procédé d'isomérisation du squelette et de la position de la double liaison des oléfines en $C_4$, utilisant un solide à base d'alumine ayant subi une mise en forme particulière en présence d'un polyorganosiloxane. Cette réaction est utilisée dans un procédé où de l'eau est co-introduite avec la charge oléfinique afin de limiter les réactions parasites. Les tests donnés en exemple indiquent des performances obtenues à des températures supérieures à 400°C après 1 h de fonctionnement, ce qui est relativement court et peut laisser penser que la stabilité du catalyseur dans le temps reste perfectible. La réaction d'isomérisation squelettale étant toujours accompagnée de réactions secondaires indésirables, une perte d'activité du catalyseur peut être expliquée par des réactions de cokage.

Les travaux décrits dans Applied Catalysis A : General, 2001, 214, 251, portent sur la déshydratation et l'isomérisation squelettale et de position de la double liaison simultanées, sur des alcools en $C_4$. Dans ces travaux, des alumines gamma éventuellement activées à l'acide sulfurique ont été utilisées à très faible GHSV (Gas Hourly Space Velocity : débit massique de charge par volume de catalyseur par heure) avec différents monoalcools comprenant une chaine aliphatique à 4 carbones pour évaluer leur sélectivité en termes de production d'oléfines correspondantes et en autres produits non désirés tels que le méthane, l'éthane, l'éthylène, le propane, le propylène, et les produits $C_5^+$. Il est ainsi démontré qu'une alumine activée à l'acide sulfurique est plus active et plus sélective qu'une alumine d'origine commerciale pour l'application combinée visée, à savoir la déshydratation conjuguée à l'isomérisation du squelette et de la position de la double liaison des oléfines résultantes. D'après ce document, il semble donc qu'une alumine la plus acide possible soit nécessaire pour réaliser conjointement la déshydratation et l'isomérisation des monoalcools en $C_4$. Les sélectivités en butènes linéaires à partir d'isobutanol (isomérisation squeletalle) ne sont cependant pas améliorées par l'activation de l'alumine avec de l'acide sulfurique. Aucune mention n'est faite de la stabilité des performances de ce solide au cours du temps sous charge. A noter que, dans une publication de la même équipe, Applied Catalysis A, 2000, 203, 5, l'activité en isomérisation squelettale de n-butènes, à partir d'une alumine activée à l'acide sulfurique préparée selon le même protocole, n'était maintenue que 5 à 6 h, ces solides nécessitant une régénération sous air toutes les 6 h.

Chadwick et al. décrivent dans Applied Catalysis A, 2001, 403, 1, la déshydratation et l'isomérisation en une étape du n-butanol pour former de l'isobutène sur zéolithes acides commerciales non modifiées. Ils comparent notamment les performances de zéolithes de taille moyenne de canaux (10MR) telles que Théta-1, ZSM-23, ZSM-5, Ferrierite, SAPO-11 et Y, les zéolithes les plus stables étant les monodimensionnelles (Theta-1 et ZSM-23), la Ferrierite se désactivant et la SAPO-11 se désaluminant.

A noter que la grande majorité des publications portaient jusque là sur la production d'isobutène à partir de butanols linéaires, l'isobutène étant une molécule clé en pétrochimie et pour la synthèse d'additifs essence tels que l'ETBE et le MTBE. De plus, les butanols linéaires étaient plus facilement produits par voies fermentaires classiques (ABE) que l'isobutanol. De récents développements ont cependant permis d'améliorer fortement les rendements en isobutanol, rendant cette charge accessible et disponible à coût attractif.

La demande de brevet WO 2011/113834 A1 décrit un procédé de déshydratation et d'isomérisation squelettale simultanées de l'isobutanol en vue de produire les oléfines correspondantes sur catalyseurs silicates cristallins, déaluminisés ou non, modifiés au phosphore ou non, du groupe FER, MWW, EUO, MFS, ZSM-48, MTT, MFI, MEL ou TON ayant un ratio Si/Al supérieur à 10, tamis moléculaires silicoaluminophosphates du groupe AEL, ou silice-, zircone-, titane- ou fluor-alumine. Dans les exemples, les auteurs mettent en évidence une très bonne sélectivité en isomères de l'isobutène à forte WHSV (masse de charge par masse de catalyseur par heure) sur zéolithe H-FER, et sur HZSM-5 modifiée au phosphore, la ZSM-5 modifiée entrainant la formation de 10% maximum de composés lourds ($C_5^+$). Aucune notion de stabilité de ces performances n'est évoquée dans ce document. Le seul autre catalyseur exemplifié est l'alumine gamma.

La déshydratation d'alcools en $C_4$ sur solides acides s'accompagne généralement de l'isomérisation de position de l'alcène formé. Ces deux réactions sont en effet concomitantes, puisque l'isomérisation de position de la double liaison de l'alcène est aussi rapide que la réaction de déshydratation du monoalcool en $C_4$. Dans le cas de l'isobutanol, l'isobutène formé initialement se protonne facilement (formation d'un carbocation tertiaire) et peut ensuite subir des réactions secondaires, notamment de dimérisation, puis de cyclisation, risquant d'entraîner la formation de produits secondaires non désirés.

## Objet et intérêt de l'invention

La présente invention a pour objet un procédé de déshydratation et d'isomérisation squelettale simultanées d'une charge comprenant au moins un monoalcool $C_4$ et contenant entre 0,5 et 50% poids d'eau en vue de produire des alcènes en $C_4$ dans lequel on met en oeuvre un catalyseur comprenant au moins un solide de type aluminosilicate non zéolithique présentant des propriétés texturales et d'acidité particulières obtenues par le mode particulier de préparation dudit solide. Le procédé mettant en oeuvre ledit catalyseur permet d'avoir une durée de cycle du catalyseur, une activité et une sélectivité en butènes linéaires dans le temps améliorées ainsi qu'une limitation des réactions d'oligomérisation par rapport aux procédés de l'art antérieur.

## Description détaillée de l'invention

La présente invention a pour objet un procédé de déshydratation et d'isomérisation squelettale simultanées d'une charge comprenant au moins un monoalcool en $C_4$ et contenant entre 0,5 et 50% d'eau, en vue de produire des alcènes en $C_4$, ledit procédé opérant à une température comprise entre 250 et 550 °C, sous une pression comprise entre à 0,1 et 1 MPa, avec une vitesse spatiale horaire comprise entre 0,1 et 10 h$^{-1}$, caractérisé en ce qu'il met en oeuvre un catalyseur comprenant au moins un solide de type aluminosilicate non zéolithique, la teneur massique en silice dudit solide étant comprise entre 4 et 95% poids dudit solide, ledit solide présentant les caractéristiques suivantes :

- un volume poreux total, mesuré par intrusion au porosimètre à mercure, compris entre 0,1 ml/g et 0,7 ml/g,
- un volume poreux total, mesuré par isotherme d'adsorption à l'azote, compris entre 0,1 ml/g et 0,7 ml/g,
- une surface spécifique BET comprise entre 100 et 550 m$^2$/g,
- un volume poreux, mesuré par intrusion au porosimètre à mercure, compris dans les pores de diamètre supérieur à 140 Å inférieur à 0,1 ml/g,
- un volume poreux, mesuré par intrusion au porosimètre à mercure, compris dans les pores de diamètre supérieur à 500 Å inférieur à 0,1 ml/g,
- un diamètre poreux moyen compris entre 20 et 140 Å,

ledit solide étant préparé selon un procédé comprenant au moins une étape a) de mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur dudit catalyseur, une étape b) de mise en forme dudit solide précurseur, une étape c) de traitement thermique et/ou hydrothermique, ladite étape c) étant réalisée avant ou après ladite étape b).

On entend par vitesse spatiale horaire (désignée également par le terme VVH) le ratio du débit volumique de charge en m$^3$/h à 15°C, 101.325 kPa (1 atm) par volume de catalyseur. Conformément à l'invention, la charge comprend au moins un monoalcool en $C_4$, ledit monoalcool en $C_4$ étant choisi parmi le 1-butanol, le 2-butanol, l'isobutanol et le ter-butanol, pris seul ou en mélange. De façon préférée, la charge comprend majoritairement de l'isobutanol. Par majoritairement, on entend que le ratio massique d'isobutanol sur les autres monoalcools en $C_4$ est supérieur à 50%, préférentiellement supérieur à 70%, et plus préférentiellement supérieur à 80%.

Conformément à l'invention, la charge contient de 0,5 à 50% poids d'eau. Elle peut également contenir au plus 10% poids d'impuretés liées aux modes d'obtention de la charge (azote, acides, aldéhydes, alcools non $C_4$ principalement). La présence d'eau dans le procédé, qu'elle soit introduite avec la charge, ou bien qu'elle résulte de la réaction de déshydratation de l'alcool, permet avantageusement de limiter les réactions d'oligomérisation, inévitables lors de la réaction d'isomérisation squelettale, et donc d'améliorer la sélectivité du catalyseur.

La concentration en eau dans le réacteur est ajustée entre 0,5% poids par rapport à l'alcool et jusqu'a 50% poids. Au delà de cette dilution le procédé devient énergétiquement difficilement justifiable. On pourra utiliser un dispositif du type recompression mecanique des vapeurs entre la charge et le réacteur ou bien sur les produits pour minimiser la consommation énergétique du procédé.

Conformément à l'invention, ledit procédé de deshydratation et d'isomérisation squelettale simultanées est opéré à une température comprise entre 250 et 550°C, de préférence entre 300 et 450 °C, sous une pression comprise entre à 0,1 et 1 MPa, de préférence entre 0,1 et 0,5 MPa, avec une vitesse spatiale horaire comprise entre 0,1 et 10 $h^{-1}$, de préférence entre 0,7 et 5 $h^{-1}$, et plus préférentiellement entre 2 et 4 $h^{-1}$.

L'unité réactionnelle pourvue du catalyseur peut être mise en oeuvre en lit fixe et en lit mobile, préférentiellement en lit fixe adiabatique. La réaction est réalisée en phase gazeuse à courant ascendant ou descendant, dans un plusieurs réacteurs avec un réchauffage entre chaque étape réactionnelle qui permet de compenser l'endothermicité globale. Pour réduire les pertes de charge dans le réacteur, on utilise préférentiellement des technologies de réacteur radial, où le catalyseur est placé entre des internes (grilles ou diffuseurs) verticaux, et où l'épaisseur de la couche de catalyseur traversée par le fluide réactif est minimisée sans pour autant recourir à des réacteurs avec un ratio entre diamètre et hauteur atypique (plus grand que 1). Le catalyseur est régénéré périodiquement. Dans le cas d'une utilisation en lit fixe, ladite unité réactionnelle réalise alternativement les réactions pour la production d'alcènes en $C_4$ et la régénération dudit catalyseur de manière à éliminer le coke déposé à la surface dudit catalyseur pendant lesdites réactions. Dans le cas d'une utilisation alternative en lit mobile, ledit catalyseur peut être transporté entre ladite unité réactionnelle et une unité de régénération.

Afin de réaliser au mieux conjointement la déshydratation des monoalcools en $C_4$ et la réaction d'isomérisation squelettale de l'alcène obtenu par ladite déshydratation, la présente invention revendique l'utilisation d'un catalyseur comprenant au moins un solide de type aluminosilicate non zéolithique présentant des propriétés texturales et d'acidité particulières. Par propriétés texturales et d'acidité particulières, on entend au sens de la présente invention un catalyseur à base d'un solide de type aluminosilicate non zéolithique, essentiellement mésoporeux et qui présente une acidité, essentiellement de type Brönsted, suffisante pour réaliser de façon optimale la déshydratation du monoalcool conjuguée à l'isomérisation du squelette et de la position de la double liaison des oléfines résultantes, sans pour autant être trop forte, ce qui conduirait à la formation de produits en $C_5^+$ via des réactions secondaires non désirées d'oligomérisation.

**Description du solide de type aluminosilicate non zéolithique compris dans le catalyseur utilisé selon l'invention.**

Ledit solide de type aluminosilicate est un solide de nature aluminosilicique, c'est à dire un solide oxyde inorganique à base des éléments aluminium et silicium, non zéolithique et essentiellement mésoporeux. Les caractéristiques dudit solide sont les suivantes, les pourcentages poids étant déterminés par rapport au poids total dudit solide :

- la teneur massique en silice ($SiO_2$) est comprise entre 4% et 95% poids, de préférence comprise entre 4 et 25% poids ou entre 35 et 95% poids, de manière plus préférée comprise entre 4% et 15% poids ou entre 35% et 50% poids et de manière encore plus préférée comprise entre 4% et 15% poids,
- la teneur en impuretés cationiques est généralement inférieure à 0,1% poids, de manière préférée inférieure à 0,05% poids et de manière encore plus préférée inférieure à 0,025% poids. On entend par teneur en impuretés cationiques la teneur totale en alcalins, en particulier en cations $Na^+$,
- la teneur en impuretés anioniques est généralement inférieure à 1% poids, de manière préférée inférieure à 0,5% poids et de manière encore plus préférée inférieure à 0,1% poids. Les impuretés anioniques présentes dans ledit solide de type aluminosilicate sont notamment des halogénures, en particulier des chlorures, ainsi que des sulfates et des nitrates,
- le diamètre moyen poreux, noté $D_{moyen}$, du solide, mesuré par intrusion au porosimètre à mercure, est compris entre 20 et 140 Å, de préférence entre 40 et 120 Å et de manière encore plus préférée entre 50 et 100 Å,
- le rapport entre le volume V2, mesuré par intrusion au porosimètre à mercure et occupé par les pores de diamètre compris entre le $D_{moyen}$ - 30 Å et le $D_{moyen}$ +30 Å, sur le volume poreux total, également mesuré par intrusion au porosimètre à mercure, est supérieur à 0,6, de préférence supérieur à 0,7 et de manière encore plus préférée supérieur à 0,8,

- le volume V3 occupé par les pores de diamètre supérieur à $D_{moyen}$ +30 Å, mesuré par intrusion au porosimètre à mercure, est inférieur à 0,1 ml/g, de manière préférée inférieur à 0,06 ml/g et de manière encore plus préférée inférieur à 0,04 ml/g,

- le rapport entre le volume V5, mesuré par intrusion au porosimètre à mercure et occupé par les pores de diamètre compris entre le $D_{moyen}$ - 15 Å et le $D_{moyen}$ +15 Å, sur le volume V2 ci-dessus, également mesuré par intrusion au porosimètre à mercure, est supérieur à 0,6, de préférence supérieur à 0,7 et de manière encore plus préférée supérieur à 0,8,

- le volume V6 occupé par les pores de diamètre supérieur à $D_{moyen}$ +15 Å, mesuré par intrusion au porosimètre à mercure, est inférieur à 0,2 ml/g, de manière préférée inférieur à 0,1 ml/g et de manière encore plus préférée inférieur à 0,05 ml/g,

- le volume poreux total, mesuré par intrusion au porosimètre à mercure, est compris entre 0,1 ml/g et 0,7 ml/g, de manière préférée compris entre 0,1 ml/g et 0,6 ml/g et de manière encore plus préférée compris entre 0,1 ml/g et 0,5 ml/g,

- le volume poreux total, mesuré par isotherme d'adsorption à l'azote, est compris entre 0,1 ml/g et 0,7 ml/g, de manière préférée compris entre 0,1 ml/g et 0,6 ml/g et de manière encore plus préférée compris entre 0,1 ml/g et 0,5 ml/g,

- la surface spécifique BET est comprise entre 100 et 550 m$^2$/g, de préférence comprise entre 150 et 500 m$^2$/g, de manière plus préférée comprise entre 150 et 350 m$^2$/g et de manière encore plus préférée comprise entre 150 et 250 m$^2$/g,

- la surface d'adsorption, laquelle est définie à partir de la branche de l'hystérésis à l'adsorption de l'azote de l'isotherme pour des pores de diamètre compris entre 3 et 200 nm, est telle que le rapport entre la surface d'adsorption et la surface BET soit supérieur à 0,5, de manière préférée supérieur à 0,65 et de manière plus préférée supérieur à 0,8,

- le volume poreux, mesuré par intrusion au porosimètre à mercure, compris dans les pores de diamètre supérieur à 140 Å est inférieur à 0,1 ml/g, de préférence inférieur à 0,05 ml/g et de manière encore plus préférée inférieur à 0,03 ml/g,

- le volume poreux, mesuré par intrusion au porosimètre à mercure, compris dans les pores de diamètre supérieur à 160 Å est inférieur à 0,1 ml/g, de préférence inférieur à 0,05 ml/g et de manière encore plus préférée inférieur à 0,025 ml/g,

- le volume poreux, mesuré par intrusion au porosimètre à mercure, compris dans les pores de diamètre supérieur à 200 Å est inférieur à 0,1 ml/g, de préférence inférieur à 0,075 ml/g et de manière encore plus préférée inférieur à 0,05 ml/g,

- le volume poreux, mesuré par intrusion au porosimètre à mercure, compris dans les pores de diamètre supérieur à 500 Å est inférieur à 0,1 ml/g, de préférence inférieur à 0,05 ml/g, de manière plus préférée inférieur à 0,02 ml/g et de manière encore plus préférée inférieur à 0,01 ml/g.

Le solide de type aluminosilicate non zéolithique est essentiellement mésoporeux, c'est à dire que le volume mésoporeux représente au moins 85% du volume poreux total, de façon préférée au moins 90% du volume poreux total et de façon encore plus préférée au moins 97% du volume poreux total.

Les données texturales données ci-dessus, caractérisant ledit solide de type aluminosilicate non zéolithique, sont déterminées par isotherme d'adsorption d'azote et par intrusion au porosimètre à mercure. L'analyse isotherme d'adsorption d'azote correspondant à l'adsorption physique de molécules d'azote dans la porosité dudit solide via une augmentation progressive de la pression à température constante renseigne sur les caractéristiques texturales (diamètre de pores, type de porosité, surface spécifique) particulières du solide de type aluminosilicate non zéolithique compris dans le catalyseur utilisé selon l'invention. En particulier, elle permet d'accéder à la surface spécifique et à la distribution mésoporeuse dudit solide. On entend par surface spécifique, la surface spécifique BET ($S_{BET}$ en m$^2$/g) déterminée par adsorption d'azote conformément à la norme ASTM D 3663-78 établie à partir de la méthode BRUNAUER-EMMETT-TELLER décrite dans le périodique "The Journal of American Society", 1938, 60, 309. La distribution poreuse représentative d'une population de mésopores centrée dans une gamme de 1,5 à 50 nm est déterminée par le modèle Barrett-Joyner-Halenda (BJH). L'isotherme d'adsorption - désorption d'azote selon le modèle BJH est décrit dans le périodique "The Journal of American Society", 1951, 73, 373, écrit par E. P. Barrett, L. G. Joyner et P. P. Halenda. Dans l'exposé ci-dessus, le "volume poreux total" correspond au volume mesuré pour P/P$_0$ = 0,99, pression pour laquelle il est admis que l'azote a rempli tous les pores. Pour finir, la dénomination "surface adsorption" se rapporte à la surface mesurée sur la branche de l'isotherme d'adsorption. On se reportera par exemple à l'article de A. Lecloux dans "Mémoires de la Société Royale des Sciences de Liège", 1971, 6ème série, Tome I, fasc.4, 169. Dans l'exposé ci-dessus, le "volume poreux" correspond au volume mesuré par intrusion au porosimètre à mercure selon la norme ASTM D4284-83 à une pression maximale de 4000 bar, utilisant une tension de surface de 484 dyne/cm et un angle de contact pour le solide de type aluminosilicate non zéolithique compris dans le catalyseur utilisé selon l'invention de 140°. Le diamètre moyen mercure est défini comme étant un diamètre tel que tous les pores de taille inférieure à ce diamètre constituent 50% du volume poreux ($V_{Hg}$), dans un intervalle compris entre 36 Å et 1000 Å. L'angle de mouillage a été pris égal à 140° en suivant les recommandations de l'ouvrage "Techniques de l'ingénieur, traité analyse et caractérisation", 1050, de J. Charpin et B. Rasneur. Afin d'obtenir une meilleure précision, la valeur du volume mercure en ml/g donnée dans le texte qui suit correspond à la valeur du volume mercure total en ml/g mesurée sur l'échantillon moins la valeur du volume mercure en ml/g mesurée sur le même échantillon pour une pression correspondant à 30 psi (environ 2 bar). Afin de mieux caractériser la distribution poreuse résultante de l'analyse par intrusion au mercure, les critères de distribution poreuse suivants sont définis : le volume V2 correspond au volume contenu dans les pores de diamètre supérieur ou égal au diamètre moyen moins 30 Å et inférieur ou égal au diamètre moyen plus 30 Å, le volume V3 correspond au volume contenu dans les pores de diamètre supérieur ou égal au diamètre moyen plus 30 Å, le volume V5 correspond au volume contenu dans les pores de diamètre supérieur ou égal au diamètre moyen moins 15 Å et inférieur ou égal au diamètre moyen plus 15 Å et le volume V6 correspond au volume contenu dans les pores de diamètre supérieur ou égal au diamètre moyen plus 15 Å.

La densité de remplissage tassée dudit solide est supérieure à 0,40 g/cm$^3$, de manière préférée supérieure à 0,45 g/cm$^3$, de manière très préférée supérieure à 0,50 g/cm$^3$.

Ledit solide de type aluminosilicate non zéolithique est de préférence un aluminosilicate homogène à l'échelle du micromètre, voir à l'échelle du nanomètre. On entend par homogène un solide aluminosilicate constitué d'une seule zone alumine-silice, ladite zone ayant en tout point un rapport Si/Al local (mesurée par exemple par analyse EDX (Energy Dispersive spectroscopy ray-X) couplée à la MET) égal au rapport Si/Al global déterminé par fluorescence X.

Ledit solide de type aluminosilicate non zéolithique présente une acidité, essentiellement de type Brönsted, suffisante pour réaliser de façon optimale la déshydratation du monoalcool conjuguée à l'isomérisation du squelette et de la position de la double liaison des oléfines résultantes sans pour autant être trop forte, ce qui conduirait à la formation de produits en $C_5+$ via des réactions secondaires non désirées d'oligomérisation.

L'acidité dudit solide de type aluminosilicate non zéolithique est avantageusement déterminée par la réalisation d'un test catalytique d'isomérisation du m-xylène en o- et p-xylène. Cette réaction d'isomérisation nécessite en effet la présence de sites acides de type Brönsted et est classiquement utilisée pour évaluer l'acidité de type Brönsted de certains solides oxydes, dont les solides de type aluminosilicate (Morin et al., J. Catal., 1996, 159, 296). Cette réaction est conduite à 350 °C sous atmosphère inerte (flux d'azote). Le m-xylène est introduit à une PPH de l'ordre de 0,25 h$^{-1}$ (débit massique de charge par g de catalyseur) dans le milieu réactionnel et traverse un lit fixe composé d'un mélange d'extrudés ou de poudre du catalyseur et de carbure de silicium. L'analyse des produits de réaction se fait par chromatographie gazeuse, la valeur des aires de chacun des constituants permet le calcul de la conversion pour un intervalle de temps donné avec A = aire du pic en $\mu$V.min :

$$\text{conversion} = \frac{\sum A_{produits}}{A_{réactifs} + \sum A_{produits}}$$

La détermination de la conversion permet le calcul de l'activité à isomasse en mmol.h$^{-1}$.g$_{solide}^{-1}$ dudit solide de type aluminosilicate pour un intervalle de temps donné avec M = masse molaire en g.mol$^{-1}$ et m = masse en g :

$$\text{activité} = \frac{\text{conversion} \times \text{débit}_{xylène} \times \text{densité}_{xylène}}{M_{xylène} \times m_{catalyseur}}$$

Pour permettre une meilleure comparaison des échantillons entre eux, la valeur de l'activité choisie est celle obtenue au bout de dix minutes, c'est-à-dire la période durant laquelle le solide de type aluminosilicate est le plus actif. Dans le cadre de l'invention, les propriétés d'acidité dudit solide de type aluminosilicate non zéolithique qui conduisent à la réalisation optimale de la déshydratation du monoalcool conjuguée à l'isomérisation du squelette et de la position de la double liaison des oléfines résultantes, sont telles que la valeur de l'activité catalytique en test d'isomérisation du m-xylène est comprise entre 0,02 mmol.h$^{-1}$g$_{solide}^{-1}$ et 0,4 mmol.h$^{-1}$g$_{solide}^{-1}$, de préférence entre 0,02 mmol.h$^{-1}$g$_{solide}^{-1}$ et 0,33 mmol.h$^{-1}$g$_{solide}^{-1}$, de façon encore plus préférée entre 0,05 mmol.h$^{-1}$g$_{solide}^{-1}$ et 0,20 mmol.h$^{-1}$g$_{solide}^{-1}$ et de façon encore plus préférée entre 0,05 mmol.h$^{-1}$g$_{solide}^{-1}$ et 0,15 mmol.h$^{-1}$g$_{solide}^{-1}$.

La composition globale dudit solide de type aluminosilicate non zéolithique, et en particulier la teneur en élément sodium, peut être déterminée par Fluorescence X (FX) sur ledit solide à l'état pulvérulent ou par Absorption Atomique (AA) après attaque acide dudit solide.

**EP 2 831 020 B1**

**Procédé de préparation du solide de type aluminosilicate non zéolithique compris dans le catalyseur utilisé selon l'invention.**

Conformément à l'invention, ledit procédé de préparation comprend au moins une étape a) de mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur audit solide de type aluminosilicate compris dans le catalyseur utilisé selon l'invention, une étape b) de mise en forme dudit solide précurseur, une étape c) de traitement thermique et/ou hydrothermique, ladite étape c) étant réalisée avant ou après ladite étape b).

La propriété de dissolution totale dans le mélange réactionnel dudit composé silicique ou desdits composés silicique et aluminique formant ladite combinaison a été évaluée de manière approchée selon la méthode suivante. Une quantité fixée (15 g) du composé silicique ou de ladite combinaison, préférentiellement hydratée, est introduite dans un milieu aqueux de pH préétabli. De manière préférée, la concentration de solide, à savoir de composé silicique ou de composés silicique et aluminique, rapporté par litre de suspension est de 0,2 mole par litre. Le pH de la solution est au moins de 12 et il peut être obtenu par utilisation d'une source alcaline. De manière préférée, il est intéressant d'utiliser NaOH. Le mélange est ensuite agité mécaniquement par un agitateur à turbine de type défloculeuse pendant 30 minutes à 800 t/min. Une fois l'agitation terminée, le mélange est centrifugé 10 minutes à 3000 t/min. Le gâteau est séparé du liquide surnageant : la solution est filtrée sur un filtre de porosité 4 de diamètre 19 cm. Le séchage et la calcination à 1000°C des 2 fractions, à savoir celle du gâteau et celle du liquide surnageant, sont ensuite réalisés. Le rapport R obtenu en divisant la masse du solide équivalent au gâteau par la masse de solide présent dans le liquide surnageant est alors défini. Par totalement soluble, on entend un rapport R supérieur ou égal à 0,9.

Par "partiellement soluble en milieu acide", il est entendu que la mise en contact dudit composé aluminique avec une solution acide, par exemple d'acide nitrique ou d'acide sulfurique, provoque sa dissolution partielle avant toute addition soit d'au moins un composé silicique totalement soluble dans le mélange réactionnel soit d'une combinaison formée d'au moins un composé silicique et d'au moins un composé aluminique, lesdits composés silicique et aluminique étant totalement solubles dans le mélange réactionnel.

Ladite propriété de dissolution partielle dudit composé aluminique a été évaluée de manière approchée selon la méthode suivante. Une quantité précise du composé aluminique en poudre ou en suspension est introduite dans un milieu aqueux de pH préétabli. Le mélange est ensuite agité mécaniquement. Une fois l'agitation terminée, le mélange est laissé sans agitation durant 24 heures. De manière préférée, la concentration du solide introduit exprimée en mol de $Al_2O_3$ par litre de suspension est de 0,5 mole par litre. Le pH de la solution de la suspension est de 2 et est obtenu soit par utilisation de $HNO_3$, soit par utilisation de HCl, soit par utilisation de $HClO_4$. De manière préférée, il est intéressant d'utiliser $HNO_3$. La répartition de l'aluminium est telle qu'une première partie de l'aluminium est présente dans une fraction sédimentée et qu'une deuxième partie de l'aluminium est présente dans une fraction dissoute. La répartition de l'aluminium dans chacune de ces deux fractions a été suivie par dosage de l'aluminium par absorption UV. La fraction dissoute encore appelée surnageant a été ultrafiltrée (membrane de polyéther-sulfones, Millipore NMWL 30000) et digérée dans de l'acide concentré. La quantité d'aluminium dans ladite fraction dissoute (surnageant) correspond au composé aluminique non-sédimenté et à l'aluminium dissous et la fraction ultrafiltrée correspond à l'aluminium dissous uniquement. La quantité de particules sédimentées est déduite de la concentration théorique en aluminium dans la suspension (en considérant que tout le solide introduit est en suspension) et des quantités d'aluminium non-sédimentées en suspension et d'aluminium dissous en solution. La présence de particules sédimentées caractérise la propriété de dissolution partielle. Dès que des particules sédimentées sont observées à l'oeil nu, le composé aluminique est dit "partiellement soluble". Les précurseurs aluminiques utilisés pour leur propriété de dissolution partielle dans la préparation du solide de type aluminosilicate non zéolithique compris dans le catalyseur utilisé dans le procédé de la présente invention se distinguent donc de ceux utilisés dans le cas des coprécipitations vraies, qui sont entièrement solubles en milieu acide, comme les sels cationiques d'alumine dont le nitrate d'aluminium par exemple, ou en milieu basique. Les méthodes utilisant lesdits précurseurs aluminiques utilisés pour leur propriété de dissolution partielle se distinguent des coprécipitations vraies car l'un des éléments, en l'espèce le composé aluminique, est partiellement soluble. Par coprécipitation vraie, on entend un procédé par lequel au moins un composé aluminique et au moins un composé silicique totalement solubles en milieu basique ou acide, sont mis en contact, simultanément ou séquentiellement, en présence d'au moins un composé précipitant et/ou coprécipitant comme décrits dans les brevets US 2.908.635, US 3.423.332, US 3.433.747, US 3.451.947, US 3.629.152 et US 3.650.988.

Cette propriété de dissolution partielle est une propriété recherchée pour la préparation du solide de type aluminosilicate non zéolithique compris dans le catalyseur utilisé dans le procédé de l'invention.

La demanderesse a découvert que le catalyseur comprenant un solide de type aluminosilicate non zéolithique obtenu selon un procédé de préparation comprenant au moins ladite étape a), une étape b) de mise en forme dudit solide précurseur, une étape c) de traitement thermique et/ou hydrothermique, ladite étape c) étant réalisée avant ou après ladite étape b), permet d'obtenir un catalyseur particulièrement performant pour la mise en oeuvre du procédé de production d'alcènes en $C_4$ selon l'invention. En effet, la mise en présence d'au moins un composé aluminique partiellement soluble en milieu acide avec soit au moins un composé silicique totalement soluble dans le mélange réactionnel soit une combinaison formée d'au moins un composé silicique et d'au moins un composé aluminique lesquels sont entièrement solubles dans le mélange réactionnel, correspond à la mise en présence d'espèces aluminiques et siliciques de taille et de réactivité chimique spécifiques conduisant de ce fait à des interactions contrôlées entre ces espèces à l'origine, pour partie, de l'homogénéité à l'échelle du micromètre, voir à l'échelle du nanomètre, du solide de type aluminosilicate compris dans le catalyseur utilisé pour la mise en oeuvre du procédé de production d'alcènes en $C_4$ selon l'invention.

En fonction de la nature chimique des composés aluminiques et siliciques utilisés pour la préparation dudit solide, le contrôle du degré d'interactivité entre les espèces siliciques et aluminiques peut être réalisé à toutes les étapes du procédé de préparation précédant l'étape c) de traitement thermique et/ou hydrothermique. Pour exemple et de façon non exhaustive, le mélange d'un composé aluminique partiellement soluble du type hydrate d'aluminium $Al_2O_3$, $nH_2O$ (boehmite) avec un composé silicique totalement soluble de type acide orthosilicique décationisé peut se faire en milieu aqueux sous l'influence de divers paramètres opératoires de synthèse contrôlés (pH, température, etc.) ou bien le mélange d'un composé aluminique partiellement soluble du type hydrate d'aluminium $Al_2O_3$, $nH_2O$ (boehmite) avec un composé silicique totalement soluble de type solution colloïdale de silice commercial (Ludox®) peut se faire lors de l'étape de mise en forme consécutivement au travail mécanique généré lors de ce procédé de mise en forme.

**Sources de silice**

Le composé de silice utilisé dans ladite étape a) selon l'invention est choisi dans le groupe formé par l'acide silicique, les sols d'acide silicique, les silicates alcalins hydrosolubles, les sels cationiques de silicium, par exemple le métasilicate de sodium hydraté, le Ludox® sous forme ammoniacale ou sous forme alcaline, les silicates d'ammonium quaternaire, pris seul ou en mélange. Le sol de silice peut être préparé selon l'une des méthodes connues de l'Homme du métier. De manière préférée, une solution d'acide orthosilicique décationisée est préparée à partir d'un silicate alcalin hydrosoluble par échange ionique sur une résine. Dans un autre mode de réalisation, les sols de silice Nyacol® ou Nyacol® Nano technologies peuvent être utilisés. Dans un autre mode de réalisation, les poudres de silice Aerosil® ou Nyasil® peuvent être utilisées.

**Sources d'aluminosilicates totalement solubles**

Les aluminosilicates hydratées totalement solubles utilisés selon l'invention peuvent être préparés par coprécipitation vraie en conditions opératoires stationnaires maîtrisées (pH, concentration, température, temps de séjour moyen) par réaction d'une solution basique contenant le silicium, par exemple sous forme de silicate de sodium, optionnellement de l'aluminium, par exemple sous forme d'aluminate de sodium, avec une solution acide contenant au moins un sel d'aluminium par exemple le sulfate d'aluminium. Au moins un carbonate ou encore du $CO_2$ peut éventuellement être rajouté au milieu réactionnel.

**Sources d'alumine**

Les composés alumininiques utilisés selon l'invention sont partiellement solubles en milieu acide. Ils sont choisis tout ou en partie dans le groupe des composés d'alumine de formule générale $Al_2O_3$, $nH_2O$. On peut en particulier utiliser des composés hydratés d'alumine tels que : l'hydrargillite, la gibbsite, la bayerite, la boehmite, la pseudo-boehmite et les gels d'alumine amorphe ou essentiellement amorphe, préférentiellement la boehmite, la pseudo-boehmite et les gels d'alumine amorphe ou essentiellement amorphe pris seul ou en mélange. On peut également mettre en oeuvre les formes déshydratées de ces composés qui sont constitués d'alumines de transition et qui comportent au moins une des phases prises dans le groupe : rhô, khi, êta, gamma, kappa, thêta, et delta, qui se différencient essentiellement par l'organisation de leur structure cristalline. L'alumine alpha appelée communément corindon peut être incorporée dans une faible proportion dans le support.

Cette propriété de dissolution partielle est une propriété recherchée pour la préparation du solide de type aluminosilicate non zéolithique compris dans le catalyseur utilisé dans le procédé de l'invention. Elle s'applique aux poudres d'alumine hydratées, aux poudres atomisées d'alumine hydratées, aux dispersions ou suspensions d'alumine hydratée ou à l'une quelconque de leur combinaison, avant une quelconque addition d'un composé contenant tout ou en partie du silicium.

Conformément à l'invention, le solide précurseur décrit ci-dessus est mis en forme au cours d'une étape b) par toute technique connue de l'Homme du métier.

La mise en forme peut être réalisée par exemple par extrusion, par dragéification, par pastillage, par pastillage et concassage, par séchage, par atomisation, par la méthode de la coagulation en goutte (oil-drop), par granulation au plateau tournant ou par toute autre méthode bien connue de l'Homme du métier. Plus précisément, quand le solide de type aluminosilicate non zéolithique se présente sous la forme d'extrudés, on peut ajouter ou retirer de l'eau pour ajuster la viscosité de la pâte à extruder. Cette étape peut être réalisée à tout stade de l'étape de malaxage. Pour ajuster la teneur en matière solide de la pâte à extruder afin de la rendre extrudable, on peut également ajouter un composé majoritairement solide et de préférence un oxyde ou un hydrate. On utilisera de manière préférée un hydrate et de manière encore plus préférée un hydrate d'aluminium. La perte au feu de cet hydrate est supérieure à 15%. La teneur en acide ajouté au malaxage avant la mise en forme est inférieure à 30%, de préférence comprise entre 0,5 et 20% poids de la masse anhydre en silice et alumine engagée dans la synthèse. L'extrusion peut être réalisée par n'importe quel outil conventionnel, disponible commercialement. La pâte issue du malaxage est extrudée à travers une filière, par exemple à l'aide d'un piston ou d'une monovis ou double vis d'extrusion. Cette étape d'extrusion peut être réalisée par toute méthode connue de l'Homme de métier. Par ailleurs, ledit solide de type aluminosilicate peut avoir été traité ainsi qu'il est bien connu de l'Homme du métier par des additifs pour faciliter la mise en forme et/ou améliorer les propriétés mécaniques finales dudit catalyseur. A titre d'exemple d'additifs, on peut citer notamment la cellulose, la carboxyméthyl-cellulose, la carboxyéthyl-cellulose, les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc. Le contrôle de la porosité caractéristique du solide de type aluminosilicate non zéolithique compris dans le catalyseur utilisé dans le procédé de l'invention est opéré partiellement lors de cette étape de mise en forme des particules de catalyseur.

Le solide de type aluminosilicate non zéolithique compris dans le catalyseur utilisé dans le procédé de l'invention se présente sous la forme de sphères, de sphéroïdes, de pastilles ou d'extrudés, préférentiellement d'extrudés. De manière très avantageuse, ledit solide se présente sous la forme d'extrudés de diamètre compris entre 0,5 et 5 mm et plus particulièrement entre 0,7 et 2,5 mm. Les formes desdits extrudés sont cylindriques (qui peuvent être creuses ou non), cylindriques torsadés, multilobées (2, 3, 4 ou 5 lobes par exemple) ou anneaux. Les formes cylindriques et multilobées sont utilisées de manière préférée, mais toute autre forme peut être utilisée.

Ledit solide de type alumonosilicate non zéolithique a généralement une résistance à l'écrasement d'au moins 70 N/cm et de manière préférée supérieure ou égale à 100 N/cm. La résistance à l'écrasement est déterminée grâce au test d'écrasement grain grain (EGG) tel que décrit dans les normes ASTM D4179-01, ASTM D6175-03.

Conformément à l'invention, la préparation dudit solide de type aluminosilicate non zéolithique comprend une étape c) de traitement thermique et/ou hydrothermique. Ladite étape c) permet d'assurer le degré d'homogénéité à l'échelle micrométrique voire nanométrique entre les espèces aluminiques et siliciques nécessaire au développement des propriétés d'acidité et des propriétés texturales du solide de type aluminosilicate non zéolithique compris dans le catalyseur utilisé dans le procédé de production d'alcènes en $C_4$ selon l'invention.

Conformément à l'invention, ladite étape c) est réalisée avant ou après ladite étape b). L'ordre de réalisation desdites étapes b) et c) n'a pas d'influence sur les caractéristiques dudit solide de type aluminosilicate non zéolithique.

Dans le cas où l'étape c) est un traitement thermique, ladite étape c) est avantageusement un séchage ou une calcination.

Le séchage est effectué par toute technique connue de l'Homme du métier.

La calcination se fait préférentiellement en présence d'oxygène moléculaire, par exemple en effectuant un balayage d'air, à une température inférieure ou égale à 1100 °C. Ce traitement par exemple peut être effectué en lit traversé, en lit léché ou en atmosphère statique. Par exemple, le four utilisé peut être un four rotatif tournant ou un four vertical à couches traversées radiales. Les conditions de calcination, température et durée, dépendent principalement de la température maximale d'utilisation dudit solide, les conditions préférées de calcination se situant entre plus d'une heure à 200°C et moins d'une heure à 1100 °C, de préférence entre 300 et 600 °C pendant une durée de 1 à 24 heures et de façon plus préférée pendant une durée de 2 à 12 heures. La calcination peut être opérée en présence de vapeur d'eau. La calcination finale peut être éventuellement effectuée en présence d'une vapeur acide ou basique. Par exemple, la calcination peut être réalisée sous pression partielle d'ammoniaque. Les traitements hydrothermiques sont effectués par toute technique connue de l'Homme du métier. Par traitement hydrothermique, on entend la mise en contact à n'importe quel étape de l'élaboration du solide avec de l'eau en phase vapeur ou en phase liquide.

Par traitement hydrothermique, on peut entendre notamment mûrissement, steaming (traitement à la vapeur), autoclavage, calcination sous air humide, réhydratation. Sans que cela réduise la portée de l'invention, un tel traitement a pour effet de rendre mobile le composant silice. De façon préférée, le traitement hydrothermique se fait par steaming dans un four en présence de vapeur d'eau. La température pendant le steaming peut être comprise entre 300 et 1100 °C et de préférence supérieure à 700 °C pendant une période de temps comprise entre 30 minutes et 12 heures, de préférence entre 30 min et 4 h. La teneur en vapeur d'eau est supérieure à 20 g d'eau par kg d'air sec et de préférence supérieure à 40 g d'eau par kg d'air sec et de manière préférée supérieure à 100 g d'eau par kg d'air sec. Un tel traitement peut, le cas échéant, remplacer totalement ou en partie le traitement de calcination.

Ledit solide peut également être avantageusement soumis à un traitement hydrothermique en atmosphère confinée. On entend par traitement hydrothermique en atmosphère confinée un traitement par passage à l'autoclave en présence d'eau sous une température supérieure à la température ambiante. La température pendant l'autoclavage peut être comprise entre 100 et 250 °C pendant une période de temps comprise entre 30 min et 6 h de préférence entre 30 minutes et 2 h.

Le solide de type aluminosilicate non zéolithique compris dans le catalyseur utilisé dans le procédé de l'invention peut éventuellement subir un post-traitement particulier visant à ajuster le niveau d'acidité dudit solide. Ce post-traitement est un traitement hydrothermique et/ou par voie chimique habituellement utilisé pour réaliser la désalumination de solides de nature zéolithique. Cette étape éventuelle peut être effectuée par toute méthode connue de l'Homme du métier (se référer par exemple à "Hydrocracking science and technology" de J. Scherzer et A.J. Gruia, Marcel Dekker Inc., 1996).

Il est également possible d'additionner au solide de type aluminosilicate non zéolithique d'autres constituants de façon à former le catalyseur utilisé selon l'invention, ces constituants pouvant être introduits lors de ladite étape b) de mise en forme. Lesdits constituants peuvent être, de façon non exhaustive, au moins un matériau oxyde poreux choisi dans le groupe formé par l'alumine, la silice, la magnésie, les argiles, l'oxyde de titane, l'oxyde de zirconium, l'oxyde de lanthane, l'oxyde de cérium, les phosphates d'aluminium, les phosphates de bore et un mélange d'au moins deux des oxydes cités ci-dessus. Ledit matériau oxyde poreux peut également être choisi parmi les mélanges alumine-oxyde de bore, alumine-oxyde de titane, alumine-zircone et oxyde de titane-zircone. Les aluminates, par exemple les aluminates de magnésium, de calcium, de baryum, de manganèse, de fer, de cobalt, de nickel, de cuivre et de zinc, ainsi que les aluminates mixtes, par exemple ceux contenant au moins deux des métaux cités ci-dessus, sont avantageusement utilisés comme matériau oxyde poreux. On peut utiliser également les titanates, par exemple les titanates de zinc, nickel, cobalt. On peut aussi avantageusement employer des mélanges d'alumine et de silice et des mélanges d'alumine avec d'autres composés comme les éléments du groupe VIB, le phosphore, le fluor ou le bore. Il est encore possible d'employer des argiles simples, synthétiques ou naturelles de type phyllosilicate 2:1 dioctaédrique ou phyllosilicate 3:1 trioctaédrique telles que la kaolinite, l'antigorite, la chrysotile, la montmorillonnite, la beidellite, la vermiculite, le talc, l'hectorite, la saponite, la laponite. Ces argiles peuvent être éventuellement délaminées. On peut aussi avantageusement utiliser des mélanges d'alumine et d'argile et des mélanges d'aluminosilicate et d'argile. De même, il peut être envisagé d'utiliser au moins un composé choisi dans le groupe formé par la famille des tamis moléculaires de type aluminosilicate cristallisé et des zéolithes synthétiques et naturelles telles que la zéolithe A, la zéolithe Y, la zéolithe Y fluorée, la zéolithe Y contenant des terres rares, USY, VUSY, SDUSY, la zéolithe X, la zéolithe L, la zéolithe bêta, la mordénite à petits pores, la mordénite à grands pores, les zéolithes oméga, NU-10, ZSM-22, NU-85, NU-86, NU-87, NU-88, Théta-1, la Ferrierite, ZSM-5, ZSM-48, ZSM-23, ZBM-30, EU-2, EU-11, Silicalite, IM-5, IM-12 et EU-1. Parmi les zéolithes on préfère habituellement employer des zéolithes dont le rapport atome de charpente silicium/aluminium (Si/Al) est supérieur à environ 3/1. On emploie avantageusement des zéolithes de structure faujasite et en particulier les zéolithes Y stabilisées et ultrastabilisées (USY) soit sous forme au moins partiellement échangées avec des cations métalliques, par exemple des cations des métaux alcalino-terreux et/ou des cations de métaux de terres rares de numéro atomique 57 à 71 inclus, soit sous forme hydrogène (Atlas of zeolite framework types, 6th revised Edition, 2007, Ch. Baerlocher, L. B. McCusker, D. H. Oison). Enfin, on peut utiliser comme matériau oxyde poreux au moins un composé choisi dans le groupe formé par la famille des tamis moléculaires de type aluminosilicate non cristallisé tels que les silices mésoporeuses, la silicalite, les silicoaluminophosphates, les aluminophosphates, les ferrosilicates, les silicoaluminates de titane, les borosilicates, les chromosilicates et les aluminophosphates de métaux de transition (dont le cobalt). Les divers mélanges utilisant au moins deux des composés cités ci-dessus conviennent également.

De préférence, le catalyseur selon l'invention est constitué intégralement du solide de type aluminosilicate non zéolithique.

EXEMPLES

**Exemple 1 : synthèse du catalyseur à base de solide du type aluminosilicate non zéolithique utilisé pour le procédé de production d'alcènes en C4, conforme à la description faite dans la présente invention (AS1).**

La poudre d'hydroxyde d'aluminium a été préparée selon le procédé décrit dans le brevet WO 00/01617. La taille moyenne des particules d'hydroxyde d'aluminium mesurée par granulométrie laser est de 40 microns. Cette poudre est mélangée à un sol de silice issu de l'acide silicique préparé par échange sur résine décationisante, puis filtré sur résine de porosité 2. Les concentrations en sol de silice et en poudre d'hydroxyde d'aluminium sont ajustées de manière à obtenir une composition finale de 90% $Al_2O_3$ et de 10% $SiO_2$. Le solide, après filtration, est laissé séché une nuit à température ambiante. La mise en forme est réalisée par un malaxage sur un malaxeur bras en Z. L'extrusion est réalisée par passage de la pâte au travers d'une filière trilobée de 2,5 mm. Les extrudés ainsi obtenus sont séchés à 150 °C, calcinés à 450°C pendant 2 h, puis traités à la vapeur à 850 °C pendant 2 h.

Les caractéristiques du support sont les suivantes :

La composition du support en silice-alumine est de 90% $Al_2O_3$ et de 10% $SiO_2$.

La surface BET est de 220 m$^2$/g.

Le volume poreux total, mesuré par adsorption d'azote, est de 0,53 ml/g

Le volume poreux total, mesuré porosimétrie mercure, est de 0,44 ml/g.

Le diamètre poreux moyen, mesuré par porosimétrie au mercure, est de 79 Å.

Le rapport entre le volume V2, mesuré par porosimétrie au mercure, compris entre le $D_{moyen}$ - 30 Å et le $D_{moyen}$ + 30 Å sur le volume poreux total mesuré par porosimétrie au mercure est de 0,93.

Le volume V3, mesuré par porosimétrie au mercure, compris dans les pores de diamètre supérieur à $D_{moyen}$ + 30 Å est de 0,017 ml/g.

Le rapport entre le volume V5, mesuré par porosimétrie au mercure, compris entre le $D_{moyen}$ - 15 Å et le $D_{moyen}$ + 15 Å sur le volume V2 ci-dessus est de 0,91.

Le volume V6, mesuré par porosimétrie au mercure, compris dans les pores de diamètre supérieur à $D_{moyen}$ + 15 Å est de 0,02 ml/g,

Le rapport entre la surface adsorption et la surface BET est de 1.

Le volume poreux, mesuré par porosimétrie au mercure, compris dans les pores de diamètre supérieur à 140 Å est de 0,0017 ml/g,

Le volume poreux, mesuré par porosimétrie au mercure, compris dans les pores de diamètre supérieur à 160 Å est de 0,0015 ml/g,

Le volume poreux, mesuré par porosimétrie au mercure, compris dans les pores de diamètre supérieur à 200 Å est de 0,0013 ml/g,

Le volume poreux, mesuré par porosimétrie au mercure, est compris dans les pores de diamètre supérieur à 500 Å est de 0,0007 ml/g,

La teneur en sodium atomique est inférieur à 40 ppm (limite de détection de l'analyse). La teneur en soufre atomique est inférieur à 40 ppm.

La valeur de l'activité catalytique en test d'isomérisation du m-xylène est de 0,07 mmol.h$^{-1}$g$_{solide}$$^{-1}$.

**Exemple 2** : synthèse d'un catalyseur à base d'un solide aluminosilicate non zéolithique non conforme à la description faite dans la présente invention (AS2).

On prépare un support aluminosilicate non conforme à l'invention conformément à l'un des protocoles de synthèse proposés dans le brevet US 4.837.193 basé sur la coprécipitation vraie en milieu aqueux d'un précurseur aluminique et d'un précurseur silicique totalement solubles dans ledit milieu par ajout d'un composé basique. Ainsi le solide est obtenu par coprécipitation d'une solution de métasilicate de sodium avec une solution de sulfate d'aluminium en présence d'hydroxyde de sodium. Les proportions des deux solutions des précurseurs silicique et aluminique sont ajustées de manière à atteindre une composition de 70 % Al$_2$O$_3$ - 30 % SiO$_2$ sur le support final. La réaction est effectuée à une température de 60 °C avec maintien constant de la valeur du pH aux alentours de 7. La suspension ainsi obtenue est alors filtrée et le précipité lavé successivement avec de l'eau et une solution de nitrate d'ammonium puis séché à 120 °C. La poudre ainsi préparée est mise en forme par un malaxage sur un malaxeur bras en Z. L'extrusion est réalisée par passage de la pâte au travers d'une filière trilobée de 2,5 mm. Les extrudés ainsi obtenus sont séchés à 150 °C puis calcinés à 550 °C pendant 2 h.

Les caractéristiques du support SA' sont les suivantes :

La composition du support en silice-alumine est de 70% Al$_2$O$_3$ et de 30% SiO$_2$.

La surface BET est de 277 m$^2$/g.

Le volume poreux total, mesuré par adsorption d'azote, est de 0,77 ml/g.

Le diamètre poreux moyen, mesuré par porosimétrie au mercure, est de 9,5 Å.

Le rapport entre le volume V2, mesuré par porosimétrie au mercure, compris entre le $D_{moyen}$ - 30 Å et le $D_{moyen}$ + 30 Å sur le volume mercure total est de 0,85.

Le volume V3, mesuré par porosimétrie au mercure, compris dans les pores de diamètres supérieurs à $D_{moyen}$ + 30 Å est de 0,045 ml/g.

Le rapport entre le volume V5, mesuré par porosimétrie au mercure, compris entre le $D_{moyen}$ - 15 Å et le $D_{moyen}$ + 15 Å sur le volume V2 ci-dessus est de 0,88.

Le volume V6, mesuré par porosimétrie au mercure, compris dans les pores de diamètres supérieurs à $D_{moyen}$ + 15 Å est de 0,067 ml/g.

Le rapport entre la surface adsorption et la surface BET est de 0,98.

Le volume poreux, mesuré par porosimétrie au mercure, compris dans les pores de diamètre supérieurs à 140 Å est de 0,036 ml/g.

Le volume poreux, mesuré par porosimétrie au mercure, compris dans les pores de diamètre supérieurs à 160 Å est de 0,033 ml/g.

Le volume poreux, mesuré par porosimétrie au mercure, compris dans les pores de diamètre supérieurs à 200 Å est 0,025 ml/g.

Le volume poreux, mesuré par porosimétrie au mercure, est compris dans les pores de diamètre supérieurs à 500 Å est de 0,017 ml/g.

La teneur en sodium atomique est de 1200 +/- 20 ppm. La teneur en soufre atomique est non détectée.

La valeur de l'activité catalytique en test d'isomérisation du m-xylène est de 0,5 $mmol.h^{-1}g_{solide}^{-1}$.

**Mise en oeuvre du test catalytique**

Tous les catalyseurs décrits dans les exemples ci-dessus sont testés pendant environ 100 h de façon identique afin de comparer leurs performances en termes de durée de vie et d'évolution de l'activité et de la sélectivité. Pendant cette durée, deux conditions de température (300 et 400 °C) et de vvh (0,7 et 5 $h^{-1}$) sont étudiées. Un point retour à la condition initiale est effectuée en fin d'essai afin de vérifier la stabilité du catalyseur. On utilise 15 g de catalyseur sous forme d'extrudés sans aucune dilution du lit catalytique par un solide inerte.

Avant le test proprement dit, on procède à l'activation du solide à 550°C sous air durant 2 heures. Cette activation consiste en une calcination qui vise à la combustion des traces d'huile, de graisse et au séchage du catalyseur avant son utilisation. La température est ensuite abaissée à la valeur souhaitée pour effectuer le test. L'injection de la charge alcool est démarrée lorsque la température de test est atteinte.

**Exemple 3 : activité en déshydratation/isomérisation de l'isobutanol sur les catalyseurs selon les exemples 1 à 2 :**

On injecte un débit d'isobutanol commercial contenant 0,5% poids d'eau afin d'atteindre la vitesse spatiale horaire visée (0,7 ou 5 $h^{-1}$). A la sortie du réacteur, tout l'effluent est analysé par une injection en ligne par chromatographie en phase gaz. L'analyseur en ligne étant équipé d'un détecteur FID, il ne permet pas la quantification de l'hydrogène, du CO ou du $CO_2$ éventuellement formé. Cependant, les réactions de déshydrogénation possibles sont suivies grâce à l'analyse de l'isobutyraldéhyde. La méthode chromatographique utilisée permet la séparation des isomères du butène et l'identification des molécules oxygénées formées. Les conditions de réaction mises en jeu sont : une température de 300 ou 400°C, le réacteur étant isotherme, et une pression de 0,1 bars relatif et une vvh de 0,7 ou 5 $h^{-1}$.

La partie hydrocarbure de l'effluent contient majoritairement tous les isomères des butènes, et des traces de propylène et des pentènes, ainsi que des traces d'hydrocarbures en $C_1$, $C_2$, $C_3$, $C_5$ et $C_6$. Les oxygénés formés (produits minoritaires) sont : le produit de déshydrogénation (isobutyraldéhyde) et du diisobutyléther (traces). L'isobutanol non converti est aussi analysé. Les résultats sont présentés dans les tableaux 1 et 2. Seuls les composés majoritaires sont reportés ainsi que quelques espèces minoritaires représentatives des réactions secondaires ayant lieu (craquage, transfert d'hydrogène).

**Tableau 1** (selon l'exemple AS1)

| Catalyseur AS1 | Charge | Effluent | | | | |
|---|---|---|---|---|---|---|
| Temps sous charge | | 5 h | 20 h | 40 h | 70 h | Point retour 100 h |
| Température (°C) | | 300 | 300 | 400 | 400 | 300 |
| vvh ($h^{-1}$) | | 0,7 | 5 | 0,7 | 5 | 0,7 |
| % espèce | | | | | | |
| Propane | | 0.015 | 0 | 0.5 | 0.4 | 0.01 |
| Propylène | | 0 | 0 | 0 | 0 | 0 |
| Isobutane | | 0.429 | 0.3 | 0.8 | 0.3 | 0.35 |
| Butène-2-trans | | 11.2 | 10.2 | 15.5 | 14.3 | 11.1 |
| Butène-1 | | 5.8 | 4.2 | 7.6 | 7 | 5.8 |
| Isobutène | | 71 | 75.4 | 60.9 | 66 | 71.1 |
| Butène-2-cis | | 9.5 | 7.9 | 11.9 | 9.6 | 9.4 |
| Somme C5+ | | 1.75 | 1.4 | 2.75 | 2 | 1.65 |
| Isobutyraldéhyde | | 0.23 | 0.1 | 0.01 | 0 | 0.22 |

(suite)

| % espèce | | | | | | |
|---|---|---|---|---|---|---|
| Butanone | | 0.06 | 0.02 | 0 | 0.01 | 0 |
| Isobutanol | 100 | 0.1 | 0.5 | 0 | 0.02 | 0.1 |
| Somme | | 100 | 100 | 100 | 100 | 100 |
| | | | | | | |
| Conversion | | 99.9 | 99.5 | 100 | 99.8 | 99.9 |
| Sélectivité en butènes linéaires dans la coupe C4 oléfines (%) | | 27.18 | 22.82 | 36.50 | 31.89 | 27.00 |
| Sélectivité en C5+ (%) | | 1.75 | 1.41 | 2.75 | 2.00 | 1.65 |
| Sélectivité butènes (%) | | 97.5 | 97.7 | 95.9 | 96.9 | 97.4 |

**Tableau 2** (comparatif AS2)

| Catalyseur AS2 | Charge | Effluent | | | | |
|---|---|---|---|---|---|---|
| Temps sous charge | | 5h | 15h | 35h | 70h | Point retour 100h |
| Température (°C) | | 300 | 300 | 400 | 400 | 300 |
| vvh (h$^{-1}$) | | 0,7 | 5 | 0,7 | 5 | 0,7 |
| % espèce | | | | | | |
| Propane | | 0,15 | 0,1 | 0,2 | 0,17 | 0,15 |
| Propylène | | 0,01 | 0 | 0,01 | 0,02 | 0,01 |
| Isobutane | | 0,3 | 0,1 | 0,4 | 0,36 | 0,35 |
| Butène-2-trans | | 12,1 | 11,9 | 13,5 | 12,2 | 10,2 |
| Butène-1 | | 3,2 | 2,9 | 3,1 | 3 | 2,2 |
| Isobutène | | 60 | 64,5 | 51,5 | 54,5 | 65 |
| Butène-2-cis | | 8,6 | 7,5 | 9,1 | 8,9 | 7,1 |
| Somme C5+ | | 15,4 | 12,8 | 22 | 20,8 | 14,2 |
| Isobutyraldéhyde | | 0,1 | 0,05 | 0,12 | 0,15 | 0,1 |
| Butanone | | 0,01 | 0,01 | 0,01 | 0,02 | 0,01 |
| Isobutanol | 100 | 0,1 | 0,1 | 0 | 0 | 0,7 |
| Somme | | 100 | 100 | 100 | 100 | 100 |
| | | | | | | |
| Conversion | | 99,9 | 99,5 | 100 | 100 | 99,3 |
| Sélectivité en butènes linéaires dans la coupe C4 oléfines (%) | | 28,49 | 25,69 | 33,29 | 30,66 | 23,08 |
| Sélectivité en C5+ (%) | | 15,42 | 12,86 | 22,00 | 20,80 | 14,30 |
| Sélectivité butènes (%) | | 83,9 | 86,8 | 77,2 | 78,6 | 84,5 |

Le catalyseur selon l'invention présente une sélectivité en butènes linéaires améliorée à 400°C et très légèrement inférieure à 300°C par rapport à AS2. Il présente une plus grande stabilité et la sélectivité en butènes linéaires est maintenue après 100h de test alors que le solide AS2 présente une désélectivation importante. De plus, le catalyseur AS1 permet de limiter fortement les réactions secondaires entraînant la formation d'oligomères ($C_5^+$).

**Revendications**

1. Procédé de déshydratation et d'isomérisation squelettale simultanées d'une charge comprenant au moins un mo-noalcool en $C_4$ et contenant entre 0,5 et 50% poids d'eau, en vue de produire des alcènes en $C_4$, ledit procédé opérant à une température comprise entre 250 et 550 °C, sous une pression comprise entre à 0,1 et 1 MPa, avec une vitesse spatiale horaire comprise entre 0,1 et 10 h$^{-1}$, **caractérisé en ce qu'**il met en oeuvre un catalyseur comprenant au moins un solide de type aluminosilicate non zéolithique, la teneur massique en silice dudit solide étant comprise entre 4 et 95% poids dudit solide, ledit solide présentant les caractéristiques suivantes :

   - un volume poreux total, mesuré par intrusion au porosimètre à mercure, compris entre 0,1 ml/g et 0,7 ml/g,
   - un volume poreux total, mesuré par isotherme d'adsorption à l'azote, compris entre 0,1 ml/g et 0,7 ml/g,
   - la surface spécifique BET est comprise entre 100 et 550 m$^2$/g,
   - un volume poreux, mesuré par intrusion au porosimètre à mercure, compris dans les pores de diamètre supérieur à 140 Å inférieur à 0,1 ml/g,
   - un volume poreux, mesuré par intrusion au porosimètre à mercure, compris dans les pores de diamètre supérieur à 500 Å inférieur à 0,1 ml/g,
   - un diamètre poreux moyen compris entre 20 et 140 Å,

   ledit solide étant préparé selon un procédé comprenant au moins une étape a) de mélange d'au moins un composé aluminique partiellement soluble en milieu acide avec soit au moins un composé silicique totalement soluble dans le mélange réactionnel soit une combinaison formée d'au moins un composé silicique et d'au moins un composé aluminique, lesdits composés silicique et aluminique étant totalement solubles dans le mélange réactionnel, de manière à former un solide précurseur dudit catalyseur, une étape b) de mise en forme dudit solide précurseur, une étape c) de traitement thermique et/ou hydrothermique, ladite étape c) étant réalisée avant ou après ladite étape b).

2. Procédé selon la revendication 1, tel que ladite charge comprend majoritairement de l'isobutanol.

3. Procédé selon l'une des revendications 1 à 2, tel que ledit composé silice utilisé dans ladite étape a) est choisi parmi l'acide silicique, les sols d'acide silicique, les silicates alcalins hydrosolubles et les sels cationiques de silicium, pris seul ou en mélange.

4. Procédé selon l'une des revendications 1 à 3, tel que ledit composé aluminique utilsé dans ladite étape a) est choisi parmi la boehmite, la pseudo-boehmite et les gels d'alumine amorphe ou essentiellement amorphe pris seul ou en mélange.

5. Procédé selon l'une des revendications 1 à 4, tel que ledit solide de type aluminosilicate présente une teneur massique en silice comprise entre 4 et 25% poids dudit solide

6. Procédé selon la revendication 5, tel que ledit solide de type aluminosilicate présente une teneur massique en silice comprise entre 4 et 15% poids dudit solide.

7. Procédé selon l'une des revendications 1 à 4, tel que ledit solide de type aluminosilicate présente une teneur massique en silice comprise entre 35 et 95% poids dudit solide.

8. Procédé selon la revendication 7, tel que ledit solide de type aluminosilicate présente une teneur massique en silice comprise entre 35 et 50% poids dudit solide.

9. Procédé selon l'une des revendications 1 à 8, tel que ledit solide de type aluminosilicate présente une teneur massique en impuretés cationiques inférieure à 0,1% poids du catalyseur.

10. Procédé selon l'une des revendications 1 à 9, tel que ledit solide de type aluminosilicate présente une teneur massique en impuretés anionique inférieure à 1% poids du catalyseur.

11. Procédé selon l'une des revendications 1 à 10, tel que ledit solide de type aluminosilicate est homogène à l'échelle du micromètre.

12. Procédé selon l'une des revendications 1 à 11, tel que les propriétés acides dudit solide de type aluminosilicate sont telles que la valeur de l'activité catalytique en test d'isomérisation du m-xylène est comprise entre 0,02 mmol.h$^{-1}$g$_{solide}$$^{-1}$ et 0,4 mmol.h$^{-1}$g$_{solide}$$^{-1}$.

13. Procédé selon l'une des revendications 1 à 12, tel que ledit catalyseur est intégralement constitué du solide de type aluminosilicate non zéolithique.

14. Procédé selon l'une des revendications 1 à 13, tel que ledit solide de type aluminosilicate non zéolithique est mis en forme sous forme de sphères, de sphéroïdes, de pastilles ou d'extrudés.

**Patentansprüche**

1. Verfahren zur simultanen Dehydrierung und Skelettisomerisation einer Charge umfassend wenigstens einen C$_4$-Monoalkohol und enthaltend 0,5 bis 50 Gew.-% Wasser, um C$_4$-Alkene zu erzeugen, wobei das Verfahren durchgeführt wird bei einer Temperatur zwischen 250°C und 550°C, einem Druck zwischen 0,1 und 1 MPa, mit einer stündlichen Raumgeschwindigkeit zwischen 0,1 und 10h$^{-1}$, **dadurch gekennzeichnet, dass** ein Katalysator eingesetzt wird, aufweisend wenigstens einen Feststoff vom Typ nicht-zeolithisches Aluminosilikat, wobei der Massegehalt an Silizium dieses Feststoffs zwischen 4 und 95 Gew.-% des Feststoffs beträgt, wobei der Feststoff folgende charakteristischen Eigenschaften aufweist:

   - Ein durch Quecksilberporosimetrie gemessenes Gesamtporenvolumen zwischen 0,1 ml/g und 0,7 ml/g,
   - Ein durch isotherme Stickstoffadsorption gemessenes Gesamtporenvolumen zwischen 0,1 ml/g und 0,7 ml/g
   - Eine spezifische BET-Oberflläche zwischen 100 und 550 m$^2$/g,
   - Ein durch Quecksilberporosimetrie gemessenes Porenvolumen in den Poren mit einem Durchmesser oberhalb von 140 Å von weniger als 0,1 ml/g,
   - Ein durch Quecksilberporosimetrie gemessenes Porenvolumen in den Poren mit einem Durchmesser oberhalb von 500 Å von weniger als 0,1 ml/g,
   - Einen mittleren Porendurchmesser zwischen 40 und 120 Å,

   wobei der Feststoff gemäß einem Verfahren hergestellt ist, umfassend wenigstens einen Schritt a) des Mischens wenigstens einer im sauren Milieu teilweise lösbaren Aluminiumverbindung mit entweder wenigstens einer in der Reaktionsmischung vollständig löslichen Siliziumverbindung oder einer Kombination wenigstens einer Siliziumverbindung und wenigstens einer Alumiumverbindung, wobei die Siliziumverbindung und die Aluminiumverbindung in der Reaktionsmischung vollständig löslich sind, um einen festen Vorläufer des Katalysators zu bilden, einen Schritt b) der Formgebung des festen Vorläufers, einen Schritt c) der thermischen und/oder hydrothermischen Behandlung, wobei der Schritt c) vor oder nach dem Schritt b) erfolgt.

2. Verfahren gemäß Anspruch 1, bei dem die Charge überwiegend Isobutanol umfasst.

3. Verfahren nach einem der Ansprüche 1 oder 2, bei dem die im Schritt a) verwendete Siliziumverbindung ausgewählt ist aus den Kieseläuren, den Kieselsäuresolen, den wasserlöslichen alkalischen Silikaten und den kationischen Salzen des Siliziums, einzeln oder in Mischung.

4. Verfahren gemäß einem der Ansprüche 1 bis 3, bei dem die in Schritt a) verwendete Aluminiumverbindung ausgewählt ist aus dem Boehmit, dem Pseudo-Boehmit und den amorphen oder im Wesentlichen amorphen Aluminiumoxidgelen einzeln oder in Mischung.

5. Verfahren gemäß einem der Ansprüche 1 bis 4, bei dem der Feststoff vom Typ Aluminosilikat einen Massengehalt an Silizium zwischen 4 und 25 Gew.-% des Feststoffs aufweist.

6. Verfahren gemäß Anspruch 5, bei dem der Feststoff vom Typ Aluminosilikat einen Massengehalt an Silizium zwischen 4 und 15 Gew.-% des Feststoffs aufweist.

7. Verfahren gemäß einem der Ansprüche 1 bis 6, bei dem der Feststoff vom Typ Aluminosilikat einen Massengehalt an Silizium zwischen 35 und 95 Gew.-% des Feststoffs aufweist.

8. Verfahren gemäß Anspruch 7, bei dem der Feststoff vom Typ Aluminosilikat einen Massengehalt an Silizium zwischen 35 und 50 Gew.-% des Feststoffs aufweist.

9. Verfahren gemäß einem der Ansprüche 1 bis 8, bei dem der Feststoff vom Typ Aluminosilikat einen Massengehalt an kationischen Verunreinigungen von weniger als 0,1 Gew.-% des Katalysators aufweist.

10. Verfahren gemäß einem der Ansprüche 1 bis 9, bei dem der Feststoff vom Typ Aluminosilikat einen Massengehalt an anionischen Verunreinigungen von weniger als 1 Gew.-% aufweist.

11. Verfahren gemäß einem der Ansprüche 1 bis 10, bei dem der Feststoff vom Typ Aluminosilikat auf der Mikrometerskala homogen ist.

12. Verfahren gemäß einem der Ansprüche 1 bis 11, bei dem die sauren Eigenschaften des Feststoffs vom Typ Aluminosilikat derart sind, dass seine katalytische Aktivität im m-Xylol Isomerisationstest zwischen 0,02 $mmol.h^{-1}g_{Feststoff}^{-1}$ und 0,4 $mmol.h^{-1}g_{Feststoff}^{-1}$ beträgt.

13. Verfahren gemäß einem der Ansprüche 1 bis 12, bei dem der Katalysator integral durch den Feststoff vom Typ nicht-zeolithisches Aluminosilikat gebildet ist.

14. Verfahren gemäß einem der Ansprüche 1 bis 13, bei dem der Feststoff vom Typ nicht-zeolithisches Aluminosilikat als Kugel, Sphäroid, Pellet oder Extrudaten geformt ist.


## Claims

1. Process for simultaneous dehydration and skeletal isomerization of a feedstock that comprises at least one $C_4$ monoalcohol and that contains between 0.5 and 50% by weight of water, for the purpose of producing $C_4$ alkenes, with said process being performed at a temperature of between 250 and 550°C, under a pressure of between 0.1 and 1 MPa, with an hourly volumetric flow rate of between 0.1 and $10^{h-1}$, **characterized in that** it uses a catalyst that comprises at least one non-zeolitic aluminosilicate-type solid, with the silica content by mass of said solid being encompassed between 4 and 95% by weight of said solid, said solid having the following characteristics:

   - A total pore volume, measured by mercury intrusion porosimetry, encompassed between 0.1 ml/g and 0.7 ml/g,
   - A total pore volume, measured by nitrogen adsorption isotherm, encompassed between 0.1 ml/g and 0.7 ml/g,
   - A BET specific surface area is encompassed between 100 and 550 $m^2$/g,
   - A pore volume, measured by mercury intrusion porosimetry, encompassed in the pores with a diameter of greater than 140 A, of less than 0.1 ml/g,
   - A pore volume, measured by mercury intrusion porosimetry, encompassed in the pores with a diameter of greater than 500 A, of less than 0.1 ml/g,
   - A mean pore diameter of between 20 and 140 A,

   with said solid being prepared according to a process comprising at least one stage a) for mixing at least one aluminum compound that is partially soluble in acid medium with either at least one silicic compound that is totally soluble in the reaction mixture or a combination formed by at least one silicic compound and at least one aluminum compound, with said silicic and aluminum compounds being totally soluble in the reaction mixture in such a way as to form a precursor solid of said catalyst, a stage b) for shaping said precursor solid, a stage c) for heat treatment and/or hydrothermal treatment, with said stage c) being carried out before or after said stage b).

2. Process according to Claim 1, such that said feedstock for the most part comprises isobutanol.

3. Process according to one of Claims 1 to 2, such that said silicic compound used in said stage a) is selected from among silicic acid, the sols of silicic acid, the water-soluble alkaline silicates, and the cationic salts of silicon, taken by itself or in a mixture.

4. Process according to one of Claims 1 to 3, such that said aluminum compound used in said stage a) is selected from among boehmite, pseudo-boehmite and the amorphous or essentially amorphous alumina gels, taken by itself or in a mixture.

5. Process according to one of Claims 1 to 4, such that said aluminosilicate-type solid has a silica content by mass of between 4 and 25% by weight of said solid.

6. Process according to Claim 5, such that said aluminosilicate-type solid has a silica content by mass of between 4 and 15% by weight of said solid.

7. Process according to one of Claims 1 to 4, such that said aluminosilicate-type solid has a silica content by mass of between 35 and 95% by weight of said solid.

8. Process according to Claim 7, such that said aluminosilicate-type solid has a silica content by mass of between 35 and 50% by weight of said solid.

9. Process according to one of Claims 1 to 8, such that said aluminosilicate-type solid bas a cationic impurities content by mass that is less than 0.1% by weight of the catalyst.

10. Process according to one of Claims 1 to 9, such that said aluminosilicate-type solid bas an anionic impurities content by mass that is less than 1% by weight of the catalyst.

11. Process according to one of Claims 1 to 10, such that said aluminosilicate-type solid is homogenous on the micrometer scale.

12. Process according to one of Claims 1 to 11, such that the acid properties of said aluminosilicate-type solid are such that the value of the catalytic activity in the m-xylene isomerization testis between $0.02 \, mmol.h^{-1} \, g_{solid}^{-1}$ and $0.4 \, mmol.h^{-1} \, g_{solid}^{-1}$.

13. Process according to one of Claims 1 to 12, such that said catalyst integrally consists of the non-zeolitic aluminosilicate-type solid.

14. Process according to one of Claims 1 to 13, such that said non-zeolitic aluminosilicate-type solid is shaped in the form of spheres, spheroids, pellets or extrudates.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5545793 A
- WO 2011113834 A1
- US 2908635 A
- US 3423332 A
- US 3433747 A
- US 3451947 A
- US 3629152 A
- US 3650988 A
- WO 0001617 A
- US 4837193 A

### Littérature non-brevet citée dans la description

- **ADKINS et al.** *J. Am. Chem. Soc.,* 1925, vol. 47, 1163
- **MAKAROVA et al.** *J. Catal.,* 1994, vol. 149, 36
- **P. BERTEAU et al.** *Applied Catalysis,* 1991, vol. 70, 307
- *Applied Catalysis A : General,* 2001, vol. 214, 251
- *Applied Catalysis A,* 2000, vol. 203, 5
- **CHADWICK et al.** *Applied Catalysis A,* 2001, vol. 403, 1
- **BRUNAUER-EMMETT-TELLER.** *The Journal of American Society,* 1938, vol. 60, 309
- **E. P. BARRETT ; L. G. JOYNER ; P. P. HALENDA.** *The Journal of American Society,* 1951, vol. 73, 373
- **A. LECLOUX.** *Mémoires de la Société Royale des Sciences de Liège,* 1971, vol. 169
- **J. CHARPIN ; B. RASNEUR.** traité analyse et caractérisation. *Techniques de l'ingénieur,* vol. 1050
- **MORIN et al.** *J. Catal.,* 1996, vol. 159, 296
- **J. SCHERZER ; A.J. GRUIA.** Hydrocracking science and technology. Marcel Dekker Inc, 1996
- **CH. BAERLOCHER ; L. B. MCCUSKER ; D. H. OISON.** Atlas of zeolite framework types. 2007